Europäisches Patentamt

European Patent Office

Office européen des brevets

(19)

(11) Veröffentlichungsnummer: **0 268 978 B1**

(12) **EUROPÄISCHE PATENTSCHRIFT**

(45) Veröffentlichungstag der Patentschrift: **09.09.92**

(21) Anmeldenummer: **87116936.3**

(22) Anmeldetag: **17.11.87**

(51) Int. Cl.5: **G01N  33/52**, G01N 33/542, G01N 33/543

(54) Verfahren und Testträger zur Bestimmung eines Analyten.

(30) Priorität: **26.11.86 DE 3640318**

(43) Veröffentlichungstag der Anmeldung:
**01.06.88 Patentblatt  88/22**

(45) Bekanntmachung des Hinweises auf die
Patenterteilung:
**09.09.92 Patentblatt  92/37**

(84) Benannte Vertragsstaaten:
**AT BE CH DE ES FR GB GR IT LI LU NL SE**

(56) Entgegenhaltungen:
DE-A- 3 237 046
DE-A- 3 605 695

(73) Patentinhaber: **BOEHRINGER MANNHEIM GMBH**
**Sandhofer Strasse 116**
**W-6800 Mannheim 31(DE)**

(72) Erfinder: **Freitag, Helmut, Dr.**
**9115 Hague Road**
**Indianapolis, IN 46250(US)**
Erfinder: **Wilk, Hans-Erich, Dr.**
**Goldregenstr. 5**
**W-6143 Lorsch(DE)**
Erfinder: **Rothe, Anselm, Dr.**
**Tiefenklinger Weg 21**
**W-6043 Birkenau(DE)**

Rank Xerox (UK) Business Services

EP 0 268 978 B1

**Beschreibung**

Die Erfindung betrifft ein Verfahren zur Bestimmung eines Analyten aus einer Probe, insbesondere einer Körperflüssigkeit mit Hilfe von spezifisch miteinander bindungsfähigen Biomaterialien sowie einen Testträger zur Durchführung des Verfahrens.

Insbesondere richtet sich die Erfindung auf immunologische Nachweisverfahren. Diese spielen seit mehreren Jahren bei analytischen Untersuchungen, insbesondere bei der quantitativen oder qualitativen Bestimmung von Bestandteilen von Körperflüssigkeiten wie Blut oder Urin eine erhebliche Rolle. Sie zeichnen sich dadurch aus, daß sie hochspezifisch und äußerst empfindlich sind. Die Nachweisverfahren basieren auf der immunologischen Wechselwirkung zwischen dem Analyten und einem diesen Analyten mit hoher Spezifität bindenden Biomaterial. Der Analyt kann dabei entweder der gesuchte Bestandteil der Körperflüssigkeit selbst oder eine in einem vorausgehenden Schritt daraus abgeleitete Substanz sein, deren Menge für die Menge des gesuchten Bestandteils in der Probe charakteristisch ist.

Durch Markieren eines der Bindungspartner der immunologischen Bestimmung kann der Grad der Umsetzung und damit die Konzentration des zu messenden Analyten bestimmt werden. Man unterscheidet verschiedene immunologische Bestimmungsverfahren nach der gewählten Markierung. Die vorliegende Erfindung richtet sich nur auf sogenannte Enzymimmunoassays, bei denen ein Enzym als Markierung verwendet wird. Der Nachweis des Enzyms erfolgt dabei, wie dies auch bei der Enzymdiagnostik üblich ist, indem man das Enzym auf ein Substrat einwirken läßt.

Das Substrat ist dabei so gewählt, daß seine durch das Enzym katalysierte Umwandlung zu einem nachweisbaren Signal führt. Im einfachsten Fall führt die Umwandlung zu einer Änderung der Farbe des Substrats, die entweder unmittelbar visuell erkannt wird oder zur quantitativen Auswertung mit Hilfe eines entsprechenden Photometers vermessen werden kann. Der Begriff "ein Nachweissignal erzeugendes Substrat" ist aber breit dahingehend zu verstehen, daß er jeden Fall umfaßt, in dem ein irgendwie erkennbares oder meßbares Signal als Folge, ggf. auch mittelbare Folge der Einwirkung des Markierungsenzyms auf das Substrat entsteht. Beispielsweise umfaßt er Fälle, bei denen das Substrat zwar selbst keinen Farbumschlag zeigt, aber eine zu einem Farbumschlag führende Folgereaktion auslöst. Er umfaßt auch Fälle, in denen ein anderes Signal als ein Farbumschlag, beispielsweise Lumineszenz gemessen wird.

Außer den immunologischen Verfahren richtet sich die Erfindung auch auf andere analytische Verfahren, bei denen im Zuge einer quantitativen oder qualitativen Bestimmung eines Analyten zwei spezifisch miteinander bindungsfähige Substanzen, die allgemein als Bindungspartner bezeichnet werden, verwendet werden. Die Bindungspartner sind meist nicht nur miteinander bindungsfähig sondern mindestens einer der Bindungspartner macht eine spezifische Bindungsreaktion mit dem Analyten oder einem analytspezifischen Produkt einer Vorreaktion.

Bei einer besonders wichtigen Gruppe solcher Verfahren ist der eine Bindungspartner (beispielsweise ein Antikörper) enzym markiert und nicht trägerfixiert (also im Test frei beweglich). Der andere Bindungspartner (beispielsweise ein Antigen) ist trägerfixiert. Die Gesamtreaktion der analytischen Bestimmung enthält - möglicherweise nach einem oder mehreren vorausgehenden Schritten - ein Reaktionsschritt, bei dem die Bindungspartner miteinander inkubiert werden, so daß eine spezifische Bindungsreaktion zwischen ihnen abläuft, wobei nach Ablauf der spezfischen Bindungsreaktion die Menge des nicht an den trägerfixierten Bindungspartner gebundenen enzymatisch markierten Bindungspartners ein Maß für die Konzentration des Analyten ist. Diese Menge wird mit Hilfe der enzymatischen Markierung bestimmt, indem man das Markierungsenzym auf ein ein Nachweissignal erzeugendes Substrat einwirken läßt. Im folgenden werden einige wichtige Beispiele solcher Verfahren beschrieben.

Bei einer ersten Variante ist der trägerfixierte Bindungspartner ein Biomaterial, welches nicht nur mit dem enzymatisch markierten nicht trägerfixierten Bindungspartner sondern auch mit dem Analyten spezifisch bindungsfähig ist. Der enzym markierte Bindungspartner ist also ein Analogon zu dem Analyten. Beide werden gleichzeitig mit dem trägerfixierten Bindungspartnter inkubiert, wobei dieser im Unterschuß gegenüber dem enzym markierten nichtfixierten Bindungspartner vorhanden ist. Der Analyt und das enzym markierte Analyt-Analogon konkurrieren um die beschränkte Anzahl von Bindungsplätzen an dem trägerfixierten Bindungspartner. Man spricht deshalb auch von kompetetiven Tests.

Will man beispielsweise ein Antigen bestimmen, so wird dieses gleichzeitig mit einer bestimmten Menge enzymmarkierten Antigens und einer demgegenüber im Überschuß vorhandenen Menge von trägerfixierten Antikörper inkubiert, bis die immunologische Bindungsreaktion abgelaufen ist. Dabei konkurriet das Antigen aus der Probe und das enzymmarkierte Antigen aus dem Reagenz um di Bindungsplätze an dem trägerfixierten Antikörper. Je mehr nichtmarkiertes Antigen in der Probe vorhanden ist, desto weniger enzymmarkiertes Antigen wird an den trägerfixierten Antikörper gebunden. Die Menge des verbleibenden nicht gebundenen enzymmarkierten Antigens ist dann also ein Maß für die Antigenmenge in der Probe.

Bei einer anderen Variante eines solchen Verfahrens ist der nicht trägerfixierte enzymatisch markierte Bindungspartner nicht nur mit dem trägerfixierten Bindungspartner, sondern auch mit dem Analyten spezifisch bindungsfähig. Beispielsweise kann ein nicht fixierter enzymmarkierter Antikörper verwendet werden, der sowohl für ein im Test vorhandenes trägerfixiertes Antigen als auch für Antigen aus der Probe spezifisch ist. Bei einer derartigen Testzusammensetzung kann man zum einen so verfahren, daß man zunächt in einer Vorreaktion, welche dem Reaktionsschritt, bei dem der enzymatisch markierte nicht trägerfixierte Bindungspartner und der trägerfixierte Bindungspartner miteinander inkubiert werden, vorgelagert ist, den enzymatisch markierten nicht trägerfixierten Bindungspartner mit dem Analyten inkubiert. In dem zitierten Beispiel läßt man also zunächst den enzymmarkierten Antikörper mit dem Probenantigen reagieren. Dabei ist bei den bekannten Verfahren der Anitkörper im Überschuß zu der maximal möglichen Menge an Antigen in der Probe vorhanden, so daß eine der Menge des Probenantigens entsprechende Menge an Antigen-Antikörperkomplexen gebildet wird. Erst nach Ablauf dieser Vorreaktion wird das Reaktionsergebnis mit dem festphasengebundenen Antigen in Kontakt gebracht, welches üblicherweise im Überschuß gegenüber dem enzymmarkierten Antikörper vorliegt. In diesem Reaktionsschritt zwischen den beiden im Test befindlichen spezifisch miteinander bindungsfähigen Biomaterialien (enzymmarkierter freier Antikörper und trägerfixiertes Antigen) wird der im vorhergehenden Reaktionsschritt nicht komplizierte Teil des enzymmarkierten Antikörpers von dem trägerfixierten Antigen gebunden und ist somit nicht mehr frei beweglich. Damit entspricht auch bei dieser Testführung die Menge des schließlich noch frei beweglichen, d. h. nicht an den trägerfixierten Bindungspartner gebundenen enzymatisch markierten Bindungspartners, der Menge des Analyten in der Probe. Dieses Testprinzip wird auch als IEMA-Test bezeichnet.

Alternativ kann statt der zweistufigen Verfahrensweise auch hier einstufig verfahren werden, d. h. Analyt, enzymmarkierter nicht trägerfixierter Bindungspartner (enzymmarkierter Antikörper) und trägerfixierter Bindungspartner (trägerfixiertes Antigen) werden gleichzeitig inkubiert. In diesem Fall laufen die Bindungsreaktionen zwischen dem enzymmarkierten Bindungspartner und dem Analyten einerseits sowie zwischen den enzymmarkierten Bindungspartner und dem trägerfixierten Bindungspartner andererseits in Konkurrenz zueinander ab. Im Ergebnis ist auch hier die Menge des friebeweglich im Test verbleibenden enzymatisch markierten Bindungspartners ein Maß für die Konzentration des Analyten.

Über die vorerwähnten Beispiel hinaus richtet sich die vorliegende Erfindung auf jedes analytische Bestimmungsverfahren, bei dem zwei in der Testzusammensetzung vorhandene spezifisch miteinander bindungsfähige Bindungspartner so miteinander inkubiert werden, daß die nach Ablauf der spezifischen Bindungsreaktion in der freien Phase vorhandene Menge der enzymatischen Markierung als Maß für die Konzentration (bzw. bei qualitativen Testen als Maß für die Anwesentheit) eines Analyten bestimmt wird.

Da bei diesen analytischen Bestimmungsverfahren die enzymatische Aktivität in der freien Phase getrennt von der gleichzeitig vorhandenen gebundenen (trägerfixierten) Phase gemessen werden muß ist bei den üblichen Verfahren die räumliche Trennung der gebundenen und der freien Phase notwendig. Dies ist bei der praktischen Durchführung der Bestimmungen mit einem erheblichen Aufwand verbunden. Da die Genauigkeit der Analyse unmittelbar davon beeinflußt wird, mit welcher Qualität die gebundene und die freie Phase voneinander getrennt werden, wird regelmäßig eine Vielzahl von Waschschritten durchgeführt. Soweit die Bestimmungen mit Hilfe entsprechender Reagenzien von Hand durchgeführt werden, ist dies sehr zeitaufwendig. Es wurden auch bereits Geräte zur mechanisierten Durchführung derartiger Bestimmungen entwickelt. Wegen der Kompliziertheit der Vorgänge sind diese jedoch aufwendig aufgebaut und damit teuer. Ihre Handhabung erfordert geschultes Personal.

Demgegenüber gesteht bereits seit langem eine Bedürfnis nach entsprechenden sehr einfach handhabbaren Bestimmungsmethoden. Wünschenswert wäre insbesondere die Übertragung der bekannten immunologischen Methoden auf trägergebundene Tests. Derartige Testträger, die auch als sogenannte Trockentests bezeichnet werden, sind für nichtimmunologische Bestimmungen seit langem bekannt. Sie haben beispielsweise die Form der bekannten Teststreifen, bei denen der eigentliche Reaktionsbereich in Form eines Testfeldes aufgebracht ist. Eine andere Ausführungsform ist als im wesentlichen quadratische Plättchen gestaltet. Hier befindet sich der Reaktionsbereich in ebenfalls quadratischer Form in der Mitte des Plättchens und ist bei den üblichsten Ausführungsformen nach Art eines photografischen Films in einem schichtweisen Verfahren hergestellt.

Es hat bereits eine Reihe von Versuchen gegeben, Bestimmungen der hier in Rede stehenden Art, insbesondere immunologische Bestimmungen auf Testträger zu übertragen. So werden beispielsweise Teststreifen angeboten, die nur einen Teil der erwähnten Testbestandteile tragen. Diese müssen dann nacheinander und in einem bestimmten zeitlichen Rhytmus in mehrere Flüssigkeiten eingetaucht werden. Dadurch wird die gewünschte einfache Handhabung nicht erreicht.

3

In der DE-A-3237046 ist eine Vorrichtung und ein Verfahren zur Bestimmung der Anwesentheit von Antigenen mit Hilfe von Testträgern beschrieben. Die Vorrichtung weist mindestens zwei Zonen auf, von denen die eine als Substratzone und die andere als immunologische Reaktionszone bezeichnet werden kann. Durch eine bestimmte Auswahl der Bestandteile in der immunologischen Reaktionszone soll nun erreicht werden, daß nur diejenigen enzymmarkierten Liganden in die Substratzone gelangen, welche für die Gegenwart bzw. Menge des Analyten charakteristisch sind, obwohl beide Zonen ständig in Flüssigkeitsverbindung miteinander stehen, wie dies bei den verschiedenen Schichten eines mehrschichtigen Testträgers üblich ist.

In der Praxis haben sich die in der DE-A-3237046 beschriebenen Testträger nicht bewährt. Dies dürfte u. a. daran liegen, daß es nicht möglich ist, zu verhindern, daß ein Teil des Überschusses an enzymmarkiertem Antikörper bei der dort beschriebenen Anordnung unvermeidlich in die Substratzone eindringt und dadurch das Meßergebnis verfälscht.

Der vorliegenden Erfindung liegt daher die Aufgabe zugrunde, Bestimmungen der Eingangs definierten Art dahingehend zu verbessern, daß sie ohne räumliche Trennung zwischen freier und gebundener Phase ausgeführt werden können. Dadurch sollen insbesondere Testträger zur Verfügung gestellt werden, welche einfach zu handhaben sind und dennoch verläßliche Ergebnisse liefern.

Die Aufgabe wird durch die in den Ansprüchen definierte Erfindung gelöst.

Ein wesentliches Element der vorliegenden Erfindung besteht darin, daß die Nachweisreaktion zeitlich verzögert, nicht aber räumlich getrennt wird. Wie oben erwähnt, kann das Nachweissignal auf verschiedenerlei Weise erzeugt werden. In der folgenden Beschreibung wird beispielhaft von dem häufigsten Fall ausgegangen, daß nämlich das Enzymsubstrat sich unter Einwirkung des Enzyms farblich ändert. Dadurch soll aber die Allgemeinheit der möglichen Nachweisreaktionen in keiner Weise beschränkt werden.

Die zeitliche Verzögerung wird dadurch erreicht, daß ein nichtfixiertes, also im praktischen Anwendungsfall frei bewegliches kein Nachweissignal erzeugendes Substrat zugleich mit einem trägerfixierten, also nicht frei beweglichen, ein Nachweissignal erzeugenden Substrat des Markierungsenzym verwendet wird, wobei beide Substrate schon während des Ablaufs der spezifischen, insbesondere immunologischen Bindungsreaktion anwesend sind. Es ist nicht notwendig, daß die Substrate während des gesamten Ablaufs der spezifischen Bindungsreaktion gemeinsam mit den Liganden inkubiert werden. Vielmehr kann das Verfahren auch so ablaufen bzw. der Test so aufgebaut sein, daß ein Teil der Bindungsreaktion schon abgelaufen ist, bevor ein Kontakt mit den Substraten stattfindet. Wichtig ist jedoch, daß, sobald das ein Nachweissignal erzeugende Substrat mit dem Markierungsenzym in Kontakt kommt, zugleich das kein Nachweissignal erzeugende Substrat anwesend ist. Dadurch wird erreicht, daß es unschädlich ist, wenn mindestens während eines Teiles der Zeit, in der die spezifische Bindungsreaktion abläuft, dies unter gleichzeitigen Kontakt mit dem Substrat geschieht.

Der Begriff "kein Nachweissignal erzeugendes Substrat" ist dahingehend zu verstehen, daß das Substrat kein die praktische Messung störendes Signal im Vergleich zu dem ein Nachweissignal erzeugenden Substrat erzeugt. Soweit also wie erwähnt ein Farbumschlag verwendet wird, muß der Farbumschlag des "kein Nachweissignal erzeugenden", d. h. "nicht farbbildenden" Substrats unter den Meßbedingungen soviel kleiner sein, als der des entsprechenden "ein Nachweissignal erzeugenden", d. h. farbbildenden Substrats, daß das Meßergebnis im Rahmen der angestrebten Meßgenauigkeit nicht gestört wird. Bei einer visuellen Auswertung darf also keine störende Farbänderung des nicht farbbildenden Substrats im gesamten Bereich des sichtbaren Lichts vorliegen. Bei einer apparativen Auswertung ist es ausreichend, wenn das Substrat bei der Meßwellenlänge des entsprechenden Auswertegerätes keine störende farblich Änderung aufweist.

Im folgenden wird das ein Nachweissignal erzeugende (trägerfixierte) Substrat mit $S$, das kein Nachweissignal erzeugende (freie) Substrat mit $S_0$ bezeichnet. Die Gegenwart von $S_0$ führt dazu, daß das Nachweissignal von $S$ erst zeitlich verzögert einsetzt, nämlich wenn die gleichzeitig ablaufende spezifische Bindungsreaktion zwischen dem enzymatisch markierten freien Bindungspartner und dem festphasengebundenen Bindungspartner im wesentlichen abgelaufen ist. Dies wird erreicht, weil das Markierungsenzym zunächst mit sehr viel größerer Wahrscheinlichkeit mit dem freien Substrat zusammentrifft und dessen Reaktion katalysiert als mit dem festphasengebundenen Substrat. Die Reaktion von $S_0$ führt jedoch zu keinem Nachweissignal, so daß in dieser Phase noch kein Signal gemessen wird.

Die Geschwindigkeit, mit der eine enzymatisch katalysierte Reaktion eines Substrats abläuft ist abhängig von der Gesamtaktivatät des eingesetzten Enzyms, der Affinität zwischen Substrat und Enzym und der Substratmenge. Dies gilt sowohl für $S$ als auch für $S_0$. Darüberhinaus ist für die Erfindung wesentlich, daß bei gleichzeitiger Anwesentheit zweier Substrate eines Enzyms, von denen das eine festphasengebunden und das andere frei beweglich ist, die enzymatisch katalysierte Reaktion des gebundenen Substrats solange nur in einem sehr stark reduzierten Umfang stattfindet, bis das freie Substrat weitgehend verbraucht ist.

Die gewünschte Verzögerung der enzymatisch katalysierten Reaktion von S ist deshalb abhängig von der Affinität und der eingesetzten Menge $S_0$. Diese sind in Relation zur Gesamtaktivität des Markierungsenzyms in dem Test festzulegen. Je höher die Aktivität des Enzyms und je höher seine Affinität zu $S_0$ ist, desto schneller wird es verbraucht. Darüber hinaus spielt auch die Menge und Affinität von S eine Rolle für die Verzögerung, wie im folgenden noch näher erläutert wird. Insgesamt kann der Fachmann aufgrund der erfindungsgemäßen Lehre sowohl durch theoretisch Überlegungen als auch durch praktische Erprobung ermitteln, ob mit dem jeweils verwendeten $S_0$ in der verwendeten Menge eine ausreichende Verzögerung erreicht wird.

Wie erwähnt kommt es wesentlich darauf an, daß die eingesetzte Menge von $S_0$, dessen Affinität zu dem Markierungsenzym und die Gesamtaktivität des Markierungsenzyms im Test richtig aufeinander abgestimmt sind. Die Affinität hängt von dem gewählten Substrat und Markierungsenzym ab und läßt sich deswegen nur insoweit frei bestimmen, wie geeignete Substrate für das jeweilige Enzym zur Verfügung stehen. Auch die Substratmenge ist nicht unbeschränkt frei wählbar. Gemäß einer bevorzugten Ausführungsform der Erfindung wird deswegen im Fällen, in denen die durch ein bestimmtes $S_0$ erreichte Verzögerung abgekürzt werden soll, zusätzlich das Markierungsenzym in trägerfixierter Form eingesetzt. Dadurch wird die enzymatisch katalysierte Reaktion von $S_0$ beschleunigt. Die enzymatisch katalysierte Reaktion von S wird dagegen von dem trägergebundenen Enzym nicht beeinflußt, weil dieses Substrat ja ebenfalls trägergebunden ist.

Um umgekehrt die Verzögerung der Nachweisreaktion zu vergrößern, d. h. um einen langsameren Verbrauch von $S_0$ zu erreichen, kann es gemäß einer anderen bevorzugten Ausführungsform der Erfindung zweckmäßig sein, nur einen Teil des freien, ersten Bindungspartners enzymatisch zu markieren. Dadurch wird die Gesamtaktivität des Markierungsenzym im Test herabgesetzt und die enzymatisch katalysierte Reaktion von $S_0$ verzögert.

Durch die Kombination der vorerwähnten Maßmahmen kann die Verzögerungszeit, nach der die enzymatisch katalysierte Reaktion von S beginnt, in weiten Bereichen variiert werden. Dadurch läßt sie sich der jeweiligen Geschwindigkeit der spezifischen Bindungsreaktion zwischen dem ersten und zweiten Liganden gut anpassen. Die erwähnte Verzögerung wird jeweils so eingestellt, daß die spezifische Bindungsreaktion im wesentlichen abgelaufen ist, wobei der Begriff "im wesentlichen" im Hinblick auf die gewünschte Meßgenauigkeit zu verstehen ist. Soweit die spezifische Bindungsreaktion nämlich noch nicht vollständig abgelaufen ist, befindet sich das System aus den Teilnehmern der spezifischen Bindungsreaktion noch nicht im Gleichgewicht. Infolgedessen ist noch ein Teil des enzymmarkierten ersten Bindungspartners frei beweglich, der im Gleichgewicht an dem trägerfixierten zweiten Liganden gebunden wäre. Wenn in diesem Stadium schon die enzymatisch katalysierte Reaktion von S einsetzt, ergibt sich daraus ein Meßfehler. Je höher die Ansprüche an die Meßgenauigkeit sind, desto länger muß also die enzymatisch katalysierte Reaktion von S verzögert werden.

Wie weiter oben erwähnt ist die Verzögerungszeit, mit der die enzymatisch katalysierte Reaktion von S einsetzt, auch von dessen Menge und Affinität gegenüber dem Enzym abhängig. Bevorzugt hat das Enzym eine höhere Affinität gegenüber dem freien $S_0$ als gegenüber dem gebundenen S. Dadurch wird erreicht, daß die Reaktion von S nur in sehr geringem Umfang katalysiert wird, solange noch eine nennenswerte Menge von $S_0$ vorhanden ist.

Die Erfindung wird im folgenden anhand von in den Figuren dargestellten Ausführungsbeispielen näher erläutert, wobei auch weitere bevorzugte Ausführungsformen und die damit erzielten Vorteile erläutert werden. Es zeigen:

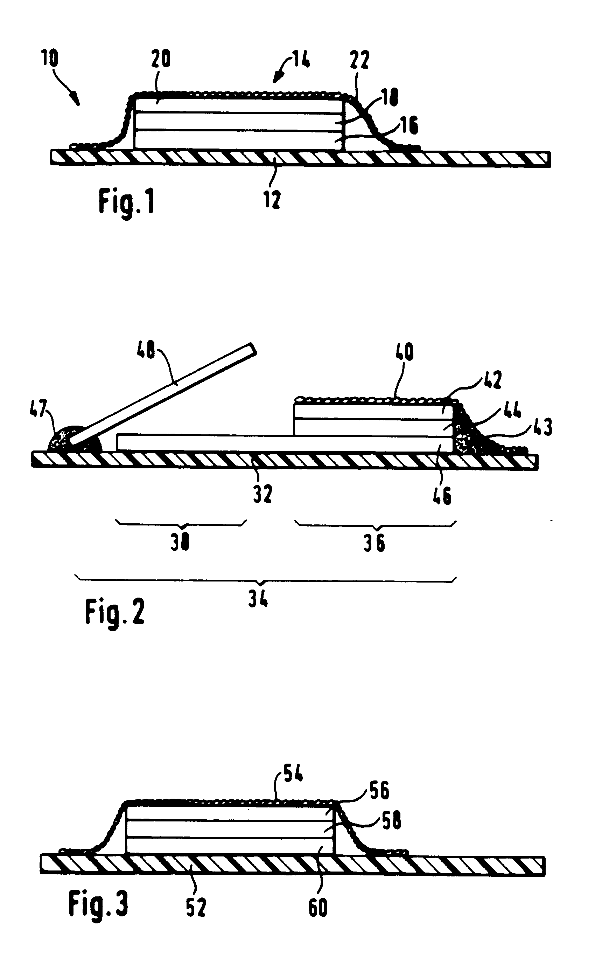

Figuren 1, 2 und 3 drei verschiedene Ausführungsformen eines erfindungsgemäßen Testträgers in einem schematisierten Querschnitt.

In Figur 1 erkennt man einen Testträger 10, der im wesentlichen aus einer langgestreckten Basisfolie 12 und dem darauf angeordneten, insgesamt mit 14 bezeichneten Reaktionsbereich besteht. Die Basisfolie 12 kann beispielsweise nach Art eines üblichen Teststreifens langgestreckt ausgebildet sein.

Der Reaktionsbereich 14 hat drei verschiedene Schichten 16, 18 und 20, welche durch ein Nylonnetz 22 auf der Basisfolie 12 festgehalten werden. Die Schichten 16, 18, 20 liegen vollflächig aufeinander auf, so daß sie in einem einen Flüssigkeitsaustausch ermöglichenden Kontakt zueinander stehen. Jede Schicht ist als beispielsweise quadratisches Plättchen von etwa 6 mm x 6 mm Fläche ausgebildet. Die Schichten werden nach den bei der Teststreifenherstellung üblichen Verfahren erzeugt und mit der Basisfolie verbunden.

Um mit der in Figur 1 dargestellten Ausführungsform eines erfindungsgemäßen Testträgers in einer flüssigen Probe enthaltenes Antigen bestimmen zu können, enthält die oberste Schicht 20 ein Konjugat aus einem für das Antigen spezifischen Antikörper und einem Markierungsenzym. Die darunter liegende Schicht 18 enthält ein nicht farbbildendes Substrat $S_0$. Die unterste Schichte 16 enthält ein trägerfixiertes farbbildendes Substrat S für das Markierungsenzym und ein trägerfixiertes Antigen, für welches der Antikörper in der Schicht 18 spezifisch ist. Weiter enthält die Schicht 16 trägerfixiertes zusätzliches Markierungsenzym, falls dies nach den oben beschriebenen Kriterien notwendig ist.

Wenn der in Figur 1 dargestellte Testträger (z. B. durch kurzes Eintauchen) mit der Probe in Kontakt gebracht wird, löst die Probenflüssigkeit das lösliche Antikörperenzymkonjugat in der Schicht 20 und das nicht farbbildende Substrat $S_0$ in der Schicht 18 auf. Da die Schichten 16, 18 und 20 in einem einen Flüssigkeitsaustausch ermöglichenden Kontakt miteinander stehen dringt das Antikörperenzymkonjugat auch in die Schichten 18 und 16 und ebenso das nicht farbbildende Substrat in die Schichten 20 und 16 ein. Insgesamt entsteht ein einheitliches gemeinsam inkubiertes Gemisch, in dem alle beschriebenen Reaktionspartner gleichzeitig anwesend sind, so daß sowohl die immunologische Bindungsreaktion als auch die enzymatisch katalysierten Reaktionen der Substrate nebeneinander ablaufen.

Das immunologische Testprinzip des in Figur 1 dargestellten Tests folgt dem oben erwähnten Prinzip, bei dem der Antikörper in der Schicht 20 spezifisch sowohl mit dem trägerfixierten Antigen in der Schicht 16 als auch mit dem Antigen in der Probe bindet. Da das Probenantigen und der Antikörper nach der Auflösung in der Probenflüssigkeit frei beweglich sind, findet eine Bindung zwischen diesen beiden Partnern zunächst mit sehr viel höherer Wahrscheinlichkeit als eine Kopplung des Antikörpers mit dem trägerfixierten Antigen in der Schicht 16 statt. Wenn der Antikörper in der Schicht 20 im Überschuß gegenüber dem Probenantigen anwesend ist, wird das in der Probe enthaltene Antigen praktisch vollständig mit dem Antikörper verbunden. Der sich bildende Komplex ist im Reaktionsbereich des Testträgers frei beweglich. Überschüssiger enzymmarkierter Antikörper bindet an das trägerfixierte Antigen in der Schicht 16 und wird dadurch fixiert.

Der Antigen-Antikörperkomplex kommt aufgrund seiner freien Beweglichkeit mit dem trägerfixierten Substrat S in der Schicht 16 in Kontakt, so daß das Markierungsenzym die Substratreaktion katalysieren kann. Der an das trägerfixierte Antigen gebundene und damit unbeweglich gewordene Überschuß des enzymmarkierten Antikörpers dagegen kann nicht mit dem ebenfalls trägerfixierten farbbildenden Substrat in Kontakt kommen und trägt deshalb zur Farbbildung nichts bei. Die Farbbildung ist daher ein Maß für Menge des Antigen-Antikörperkomplexes.

Für die Genauigkeit der Bestimmung des Antigens ist dabei wesentlich, daß zunächst die immunologische Bindungsreaktion weitgehend abläuft, so daß die Menge des gebildeten Antigen-Antikörperkomplexes der Menge des Antigens in der Probe entspricht, während andererseits der Überschuß an Antikörper nahezu vollständig an das trägerfixierte Antigen gebunden und damit dem Reaktionsgemisch entzogen sind. Würde die Farbbildungsreaktion einsetzen, bevor dieser Zustand erreicht ist, so würden sich noch enzymmarkierter Antikörper in frei beweglicher Form in dem Inkubationsgemisch befinden, der nicht mit einem Antigen komplexiert ist. Wenn dieser in Kontakt mit dem trägerfixierten Substrat käme und eine Farbbildung auslösen würde, so würde das Meßergebnis verfälscht. Dies wird durch die Verzögerung mit Hilfe des nicht farbbildenden Substrat $S_0$ vermieden.

Aufgrund der vorher dargelegten erfindungsgemäßen Relationen zwischen den eingesetzten Substraten und dem Markierungsenzym und aufgrund der Tatsache, daß das farbbildende Substrat trägerfixiert ist, wird zunächst weit überwiegend die enzymatische Reaktion von $S_0$ katalysierte, welche nicht zu einer Farbbildung führt. Erst wenn $S_0$ weitgehend aufgebraucht ist, wirkt das Markierungsenzym in verstärktem Umfang auch auf das trägerfixierte Substrat S ein und es kommt zur Farbbildung.

Den Verlauf der Farbbildung für drei verschiedene Konzentrationen an nicht farbbildendem Substrat $S_0$ ist in Tabelle 1 dargestellt. Die Tabelle basiert auf einem Beispiel, bei dem als farbbildendes Substrat S 0,1 mM Chlorphenolrotgalaktosid verwendet wurde. Sie zeigt die Farbbildung ohne $S_0$ und mit 1 mM, 5 mM bzw. 10 mM Nitrophenylgalaktosid als $S_0$ (Beta-Galaktosidase 250 mU/ml). Man erkennt deutlich, daß die Farbbildung mit umso größerer Verzögerung einsetzt, je mehr nichtfarbildendes Substrat anwesend ist.

## Tabelle 1:

| $[S_0]$ | 0mM | 1mM | 5mM | 10mM |
|---|---|---|---|---|
| | | % Farbbildung | | |
| Zeit | | | | |
| 2 Min. | 44 | 23 | 2 | 1 |
| 6 Min. | 99 | 98 | 9 | 4 |
| 10 Min. | 100 | 100 | 20 | 8 |
| 20 Min. | 100 | 100 | 96 | 20 |

Selbstverständlich gelten die angegebenen Zahlenwerte in Tabelle 1 nur für ein bestimmtes Paar $S/S_0$. Der Verlauf der Farbbildungsreaktion ist jedoch auch für andere erfindungsgemäße Zusammensetzungen ähnlich und läßt sich aufgrund der erfindungsgemäßen Lehre in verhältnismäßig breitem Umfang durch entsprechende Wahl der Reaktionsbestandteile variieren.

In der Schicht 16 kann wie erwähnt das Markierungsenzym in trägerfixierter Form inkorporiert sein. Die enzymatische Aktivität in dem Test besteht dann aus der Summe des Markierungsenzyms des Antikörpers aus der Schicht 18 und dem fixierten Enzym in der Schicht 16. Die Reaktion des nicht farbbildenden Substrats $S_0$ kann sowohl von dem trägerfixierten Enzym als auch von dem Markierungsenzym katalysiert werden. Dadurch wird das $S_0$ schneller verbraucht, als wenn kein trägerfixiertes Enzym verwendet würde. Die Verzögerungszeit bei einer gegebenen Menge $S_0$ wird damit verkürzt. Auf die Reaktion des farbbildenden Substrats hat die Anwesentheit des trägerfixierten Enzyms keinen Einfluß, weil beide Reaktionsbestandteile trägerfixiert sind und deswegen nicht in Kontakt miteinander treten können.

Die Tatsache, daß die Reaktionsbestandteile bei der in Figur 1 dargestellten bevorzugten Ausführungsform der Erfindung in drei verschiedenen Schichten des Testträgers angeordnet sind, hat vorwiegend herstellungstechnische Gründe. Bei der Herstellung der Trägerschichten werden die Reaktionsbestandteile nämlich üblicherweise auf eine feste Trägermatrix imprägniert oder in einen entsprechenden Film aus der flüssigen Phase inkorporiert. Soweit bei diesem Vorgang Reaktionsbestandteile gleichzeitig anwesend wären, welche miteinander reagieren können, müßten besondere Maßnahmen ergriffen werden, um eine solche Reaktion zu verhindern. Es ist deswegen für die Herstellung vorteilhaft, wenn einerseits der Antikörper und das Antigen und andererseits die Substrate und das Markierungsenzym in getrennten Schichten inkorporiert sind. Im konkreten Beispiel ist der Antikörper in der Schicht 20 von dem Antigen in der Schicht 16 getrennt. Außerdem ist die Enzymmarkierung in der Schicht 20 sowohl von dem nichtfarbbildenden Substrat in der Schicht 18 als auch von dem farbbildenden Substrat in der Schicht 16 getrennt. Allgemein gesprochen ist es vorteilhaft, wenn der erste Bindungspartner, welcher die Enzymmarkierung trägt in einer Schicht enthalten ist, die nicht zugleich eines der Substrate enthält und wenn der enzymatisch markierte nicht trägerfixierte Bindungspartner und der trägerfixierte Bindungspartner in getrennten Schichten enthalten sind.

Figur 2 zeigt eine Ausführungsform der Erfindung, wie sie von dem Analysesystem "Reflotron" der Anmelderin und aus zahlreichen Publikationen, beispielsweise dem Europäischen Patent 45 476 grundsätzlich bekannt ist.

Eine Besonderheit dieses Systems besteht darin, daß sich der Reaktionsbereich 34, welcher in seiner Gesamtheit wiederum von einer Basisfolie 32 getragen wird, in einen Aufgabebereich 36 und in einen Nachweisbereich 38 unterteilen läßt.

Der Aufgabebereich 36 enthält mehrere übereinander angeordnete Schichten, im dargestellten Ausführungsbeispiel unter einem Abdecknetz 40 eine Glasfaserschicht 42, darunter eine Reagenzschicht 44, welche - wiederum für das Beispiel einer Bestimmung eines Antigens - einen enzymmarkierten Antikörper in löslicher Form enthält und darunter ein Glasfaservlies 46. Die Schichten 42, 44 und 46 sind mit einem Schmelzkleberstreifen 43 befestigt.

Das Glasfaservlies 46 hat in Richtung auf den Nachweisbereich eine größere Ausdehnung als die darüber befindliche Schichten, d. h. es verbindet den Aufgabebereich 36 mit dem Nachweisbereich 38. Über dem nicht von den Schichten 42 und 44 bedeckten Teil des Glasfaservlieses 46 befindet sich eine mit einem Schmelzkleberstreifen 47 befestigte Klappe 48, die so hergestellt ist, daß sie ohne Ausübung eines zusätzlichen Druckes nicht in Kontakt mit dem Glasfaservlies 46 ist. Sie kann jedoch manuell oder mit Hilfe eines mechanischen Bestandteiles des entsprechenden Auswertegerätes nach unten auf das Glasfaservlies 46 gedrückt werden, so daß sie mit diesem in einen Flüssigkeitsaustausch ermöglichenden Kontakt tritt.

Die Klappe 48 enthält einen Reagenzfilm, auf dem sich ein lösliches nicht farbbildendes Substrat $S_0$ sowie in trägerfixierter Form ein Antigen, für das der Antikörper in der Schicht 44 spezifisch ist, und ein farbbildendes Substrat S für das Markierungsenzym des Antikörpers in der Schicht 44 sowie gegebenenfalls zusätzliches trägerfixiertes Markierungsenzym befindet.

Der Test läuft nun so ab, daß die Probe - bevorzugt Blut - in Form eines Tropfens auf den Aufgabebereich 36 aufgegeben wird. Sie dringt durch das Abdecknetz 40 in die Glasfaserschicht 42 ein, die dazu dient, die Erythrozyten im Blut abzutrennen, so daß nur Plasma durch die Glasfaserschicht 42 hindurchsickert und in die Schicht 44 gelangt, wie dies in der zitierten EP-B-45 476 näher beschrieben wird.

Die in die Schicht 44 eindringende Probe löst dort den enzymmarkierten Antikörper. Das Antigen aus der Probe wird von dem enzymmarkierten Antikörper gebunden und es entsteht Antigen-Antikörperkomplex, der enzym markiert ist. Außerdem befindet sich auch nach Ablauf dieser spezifischen Bindungsreaktion zwischen dem Probenantigen (Analyt) und dem enzymmarkierten Antikörper (nicht trägerfixierter Bindungspartner) enzymmarkierter Antikörper in der Schicht 44, der nicht mit dem Antigen gebunden ist.

Die Flüssigkeit mit den erwähnten Reaktionsbestandteilen dringt weiterhin in das Glasfaservlies 46 ein und wird von diesem aufgrund von Kapillarwirkung unter die Klappe 48 transportiert.

Ein wesentlicher Unterschied gegenüber der in Figur 1 dargestellten Ausführungsform besteht darin, daß bei der in Figur 2 dargestellten Ausführungsform der Zeitpunkt des Kontaktes zwischen der Klappe und dem Glasfaservlies frei gesteuert werden kann. Mit anderen Worten eine erste Inkubationsphase, in der die spezifische Bindungsreaktion zwischen dem Analyten und dem nicht trägerfixierten Bindungspartner abläuft kann von einer zweiten Inkubationsphase, in der die in der Klappe enthaltenen Bestandteile hinzugefügt werden, vollkommen getrennt ablaufen. Dadurch ist es möglich, die Einstellung des Gleichgewichts zwischen Analyt und freiem Bindungspartner abzuwarten, bevor die weitere Reaktion durch Herunterdrükken der Klappe 48 eingeleitet wird.

Die spezifische Bindungsreaktion zwischen dem freien enzymmarkierten und dem trägerfixierten Bindungspartner beginnt erst, wenn nach Ablauf der Vorreaktion die Klappe 48 nach unten gedrückt und dadurch in Kontakt mit dem Glasfaservlies 46 gebracht wird. Nun läuft die spezifische Bindungsreaktion zwischen dem enzymmarkierten Antikörper (freier Bindungspartner) aus der Schicht 44, soweit er nicht mit Probenantigen komplexiert ist und trägerfixiertem Antigen (trägerfixierte Bindungspartner) auf der Klappe 48 ab. Gleichzeitig katalysiert das Markierungsenzym zunächst bei weitem überwiegend die Reaktion des durch die Probenflüssigkeit aufgelösten frei beweglichen nichtfarbbildenden Substrat $S_0$, so daß die Farbbildungsreaktions des trägerfixierten Substrats S in nennenswertem Umfang erst später, nämlich zu einem Zeitpunkt auftritt, zu dem die spezifische Bindungsreaktion zwischen dem enzymmarkierten Antikörpern und dem trägerfixierten Antigen im wesentlichen abgelaufen ist. Auch in diesem Beispiel setzt daher, trotz gleichzeitiger Inkubation der beiden Bindungspartner und des farbbildenden Substrats, die Farbbildung erst zu einem Zeitpunkt ein, in dem sich nur noch so wenig nicht komplexierter enzymmarkierter Antikörper in dem Inkubationsgemisch befindet, daß er das Meßergebnis praktisch nicht mehr verfälschen kann. Die Farbreaktion weist praktisch nur noch den frei beweglichen enzymmarkierten Komplexe aus Antigen und Antikörper nach.

Bei den anhand der Figuren 1 und 2 erläuterten Testführungen ist üblicherweise der freie Bindungspartner im Überschuß gegenüber dem Analyten und der trägerfixierten Bindungspartner im Überschuß zu dem freien Bindungspartner vorhanden. Dies erfordert jedoch verhältnismäßig große Mengen der betroffen Testbestandteile. Besonders bevorzugt wird die vorliegende Erfindung mit der in der deutschen Patentanmeldung 3624464.3 (DE-A-3624464) beschriebenen Erfindung kombiniert, bei der auch geringere Mengen der Bindungspartner eingesetzt werden können.

Das in Figur 3 dargestellte Ausführungsbeispiel ähnelt in seinem Aufbau dem von Figur 1. Es unterscheidet sich jedoch in der Beschichtung der verschiedenen Schichten. Auch bei diesem Ausführungsbeispiel werden insgesamt drei Schichten von einem Nylonnetz 54 auf einer Basisfolie 52 in einem Flüssigkeitsaustausch ermöglichenden Kontakt gehalten.

Die oberste Schichte 56 enthält, wiederum für die Bestimmung eines Antigens als Analyt aus der Probe, ein Konjugat aus einem Antigen und einem Markierungsenzym. Die mittlere Schicht 58 enthält einen Antikörper, der sowohl das Antigen aus der Probe als auch das Antigen in der Schicht 56 spezifisch bindet, in trägerfixierter Form. Außerdem kann sie Markierungsenzym in trägerfixierter Form enthalten.

Die unterste Schicht 60 schließlich enthält das trägerfixierte farbbildende Substrat und das freie, nicht farbbildende Substrat.

Der in Figur 3 dargestellte Test ist vom kompetitiven Typ, d. h. der trägerfixierte Antikörper (trägerfixierter Bindungspartner) bindet spezifisch sowohl das Antigen (Analyt) aus der Probe als auch das enzymmarkierte, freie Antigen (freier Bindungspartner).

Das Probenantigen und das enzymmarkierte Antigen aus der Schicht 56 konkurrieren um Bindungsstellen an dem trägerfixierten Antikörper in der Schicht 58. Nach Ablauf dieser konkurrierenden spezifischen Bindungsreaktion ist die Menge des nicht gebundenen enzymmarkierten Antigens ein Maß für die Menge des Probenantigens, wobei umsomehr enzymmarkierte Antigen frei bleibt, je mehr Antigen in der Probe war. Auch hier ist es wider erforderlich daß die Farbbildung erst einsetzt, wenn die geschilderte immunologische Bindungsreaktion nahezu vollständig abgelaufen ist. Würde sie zu früh einsetzen, so würde das Meßergebnis verfälscht, weil die Menge des freien enzymmarkierten Antigens noch kein reproduzierbares Maß für die Menge der Probenantigene wäre. Auch in diesem Fall wird die notwendige Verzögerung der Farbbildungsreaktion, wie zuvor beschrieben, mit Hilfe des nicht farbbildenden Substrats $S_0$ in der Schicht 60 erreicht.

Es lassen sich zahlreiche Variationen der erfindungsgemäßen Prinzipien vorstellen, die von den anhand der Figuren 1 bis 3 dargestellten bevorzugten Ausführungsformen abweichen. Insbesondere richten sich diese drei Ausführungsformen jeweils auf die Bestimmung eines Antigens als Anlayt. Soll im Gegensatz hierzu ein Antikörper aus der Probe bestimmt werden, so ist der Testaufbau in jedem der Beispiele in dem Fachmann geläufiger Art und Weise so zu variieren, daß überall dort wo zuvor ein Antikörper beschrieben wurde, ein Antigen eingesetzt wird und umgekehrt. Im übrigen ist der Ablauf entsprechend.

Beispiel 1:

Teststreifen zum Nachweis von beta-hCG im Harn gemäß Figur 1

1. Testschicht 20

Fab-Anti-hCG-beta-Galactosidase-Konjugat wird aus Schaf-Anti-hCG-Serum hergestellt (Immunisierung: D.M. Weir, Handbook of Experimental Immunology , A 2.8; Fab-Spaltung: M.E. Davis, A.J. Barrett, R.M. Hernbry, J. of Immunological Methods, 21 305 (1978); Konjugation: T. Kitagawa in Enzym Immuno Assay, Editors: E. Ishikawa, T. Kawai, K. Miyai, Verlag Igaku-Shoiu, Tokio - New York 1981). und zu 5 U/ml in 10 mM K-Phosphat-Puffer, 5 mM $MgCl_2$, 25 mM Nacl, 2 % Saccharose, 0,5 % Rinderserumalbumin, 0,1 % $NaH_3$, pH 7,0 gelöst in Wasser.

Die Lösung wird auf Teebeutelpapier 212 (Schoeller & Hoesch, Gernsbach, Bundesrepublik Deutschland) getränkt und bei Raumtemperatur 30 Min. getrocknet.

2. Testschicht 16

2.1 Fixierung von Galaktosidase an Latex

1 g Latex (Serva Unisphere, 2 $\mu$m, Serva, Heidelberg, Bundesrepublik Deutschland [BRD]) mit freien Carboxylgruppen wird mit 50 ml Wasser gewaschen. Zu der Latex-Suspension in 10 ml Wasser werden 100 mg 1-Ethyl-3-(3-dimethylaminopropyl)carbodiimid. HCl (Serva Feinbiochemica) gegeben. Nach einer Stunde werden 200 mg beta-Galactosidase (Boehringer Mannheim GmbH) dazugegeben und 2 weitere Stunden inkubiert. Danach wird wiederholt mit glycinhaltigem (100 mM) Puffer (PBS) gewaschen und als Suspension bei 4°C gelagert (maximal etwa 48 Stunden). Die Aktivität der fixierten Galaktosidase wird im kolorimetrischen Test ermittelt.

2.2 Fixierung von Chlorphenolrotgalaktosid

1 g Latex wird analog zu 2.1 mit Carbodiimid aktiviert. Danach werden 10 ml 400 mM 1,6-Diaminohexan (Sigma-Chemie, Heidelberg, BRD), pH 7 % (titriert mit 1 N HCL) zugegeben und 2 weitere Stunden inkubiert. Danach wird wiederholt mit $H_2O$ gewaschen.

1 g des somit erhaltenen Crotein C-derivatisiertes Latex in 10 ml phosphatgepufferter physiologischer Kochsalzlösung (PBS ) werden mit 10 ml 25 % Glutadialdehyd versetzt und 2 Stunden bei Raumtemperatur gerührt. Danach wird abgesaugt, gewaschen und in 10 ml PBS suspendiert. Dazu werden 0,5 g 4″-Aminochlorphenolrotbeta-galaktosid gemäß der Europäischen Patentanmeldung mit der Publikationsnummer EP 0 146 866 gegeben, 6 Stunden gerührt, 30 Minuten mit 0,4 g $NaCNBH_3$ reduziert, abgesaugt, gewaschen und getrocknet.

2.3 Fixierung von beta-hCG

500 IU beta-HCG (Boehringer Mannheim GmbH) werden analog zur beta-Galaktosidase-Kopplung an Latex fixiert.

2.4 Herstellung der Reagenzschicht 16

Ca. 10 g Filmmasse werden hergestellt aus:

| | |
|---|---|
| 1 g | beta-hCG-Latex |
| 1 g | Chlorphenolrotgalaktosid-Latex |
| 0,5 g | beta-Galaktosidase-Latex (ca. 80 U) |
| 1 g | Vinylpropionat-Vinylacetat-Mischpolymerisat (Propiofan 70 D, BASF, BRD) |
| 3,5 g | 1,4 % Alginat-Lösung (Kelco, Bremen, BRD) |
| 40 mg | Tween 20 (Serva, Heidelberg, BRD) |
| 3 ml | PBS |

Die Mischung wird 0,15 mm dick auf eine 0,2 mm starke weiß pigmentierte Polycarbonat-Folie (Lonza Wereke, Waldshut, BRD) aufgezogen und 30 Minuten bei 35 ° getrocknet.

3. Testschicht 18

40 mm Nitrovinylgalaktosid in PBS werden auf Teebeutelpapier 212 (Schöller und Hoesch, Gernsblach, BRD) getränkt und bei Raumtemperatur 30 Minuten getrocknet.

4. Aufbau eines Teststreifens

Als Basisfolie 12 wird eine schmelzkleberbeschichtete Polyvinylchloridfolie verwendet. Auf der PVC-Folie wird die Testschicht 16 (Polycarbonat-Folie) darauf die Testschicht 18 und zu oberst die Testschicht 20 mit Hilfe eines Nylonnetzes (Fadendicke 0,06 mm, 45 % freie Lochfläche) befestigt. Zur Befestigung wird Schmelzkleber verwendet. Das Format der Testschichten ist 6x6 mm.

5. Bestimmung von hCG in Harn

Der Teststreifen wird für 1 Sekunde in den Probeharn eingetaucht und nach 8 Minuten visuell ausgewertet.

Dabei entstehen in Abhängigkeit von der hCG-Konzentration folgende Farbabstufufngen:

| hCG-Konzentration im Harn mU/ml | Reaktionsfarbe nach 8 Minuten |
|---|---|
| 0 | gelb |
| 100 | gelb mit leicht bräunlichem Ton |
| 400 | deutlich erkennbar rot-violett |
| 1000 | stark rot-violett |

Diese Nachweisgrenze erfüllt die Forderung nach einem Schwangerschaftsnachweis, der mit einer Empfindlichkeit von 300 U/ml eine Schwangerschaft am dritten Tag nach Ausbleiben der Monatsblutung nachweisen kann.

Beispiel 2:

Quantitativer, reflexionsphotometrisch auszuwertender Testträger für Theophyllin in Vollblut gemäß Figur 2

1. Testschicht 44

Fab-Anti-Theophyllin-beta-Galaktosidase-Konjugat wird durch Immunisierung von Schafen mit Theophyllin-8-Carboxypropyl-Edestin hergestellt. Fab-Spaltung und Konjugation mit beta-Galaktosidase erfolgen wie in Beispiel 1. Das Konjugat wird zu 100 U/ml im gleichen Puffer und wie in Beispiel 1 auf Papier getränkt.

2. Testschicht 48

2.1 Fixierung von Theophyllin

1 g Latex wird analog zu 2.1 von Beispiel 1 mit 100 mg Theopyllin-Polyhapten [Theopyllin-8-Carboxylpropyl-IGG, (8:1 = Theopyllin: IGG)] statt beta-Galaktosidase umgesetzt.

2.2 Ca. 10 g Filmmasse werden hergestellt aus:

| | |
|---|---|
| 1 g | Theophyllin-Latex |
| 1 g | Chlorphenolrotgalaktosid-Latex (hergestellt wie bei Beispiel 1) |
| 10 mM | Nitrophenylgalaktosid |
| 1 g | Vinylacetat-Vinylpropionat-Mischpolymerist (Propiofan 70 D) |
| 2,5 g | 1,4 % Alginat-Lösung |
| 40 mg | Tween 20 |
| 3 ml | PBS |

Die Mischung wird 0,15 mm dick auf eine 0,2 mm starke einseitig matte, klare Polycarbonatfolie (Lonza) aufgezogen und 30 Minuten bei 35 ° C getrocknet.

3. Aufbau des Testträgers

Der Testträger ist gemäß Figur 2 aufgebaut. Die Basisfolie 32 besteht aus 0,3 mm dickem Polystyrol. Zur Befestigung der Schichten wird Schmelzkleber verwendet.

4. Bestimmung von Theopyllin aus Vollblut

30 $\mu$l Vollblut werden auf das Glasfaservlies 42 pipettiert und der Testträger wird mit dem reflexionsphotometrischen Analysegerät "Reflotron" der Boehringer Mannheim GmbH ausgewertet. Das Gerät ist so programmiert, daß die Klappe 48 nach 2 Minuten angedrückt wird. Innerhalb dieser Zeit wurde das Plasma abgetrennt, das Antikörper-Enzym-Konjugat gelöst und die Bindungsreaktion zwischen dem Theophyllin der Probe und dem Konjugat hat stattgefunden. Das Gerät ist weiterhin so programmiert, daß 7 Minuten nach dem Andrücken der Klappe 48 die Remission gemessen wird. Folgende Meßwertabstufungen wurden erzielt:

| Theophyllin (mg/l) | % Remission |
|---|---|
| 0 | 63 |
| 1 | 62 |
| 3 | 58 |
| 10 | 42 |
| 30 | 21 |
| 45 | 18 |

Man erkennt, daß die Veränderung der Theopyllin-Konzentration im klinisch relevanten Bereich zu einer stgarken Remissionsänderung führt. Dadurch wird eine hohe Meßgenauigkeit erreicht.

**Patentansprüche**

**1.** Verfahren zur Bestimmung eines Analyten aus einer Probe, insbesondere einer Körperflüssigkeit,
bei dem zwei spezifisch miteinander bindungsfähige Biomaterialien (Bindungspartner) verwendet werden,
von denen der eine Bindungspartner enzymatisch markiert und nicht trägerfixiert und
der andere Bindungspartner trägerfixiert ist,
welches Verfahren einen Reaktionsschritt enthält, bei dem die Bindungspartner miteinander inkubiert werden, so daß eine spezifische Bindungsreaktion zwischen ihnen abläuft, wobei nach Ablauf der spezifischen Bindungsreaktion die Menge des nicht an den trägerfixierten Bindungspartner gebundenen enzymatisch markierten Bindungspartners ein Maß für die Konzentration des Analyten ist und diese Menge mit Hilfe der enzymatischen Markierung bestimmt wird, indem man das Markierungsenzym auf ein ein Nachweissignal erzeugendes Substrat einwirken läßt,
dadurch gekennzeichnet, daß
man während der spezifischen Bindungsreaktion zugleich mit einem nicht fixierten, kein Nachweissignal erzeugenden Substrat des Markierungsenzyms und mit einem trägerfixierten, ein Nachweissignal erzeugenden Substrat des Markierungsenzyms inkubiert,
wobei das kein Nachweissignal erzeugende Substrat hinsichtlich eingesetzter Menge und Affinität zu dem Markierungsenzym in Relation zur Menge des ein Nachweissignal erzeugenden Substrats und dessen Affinität zum Markierungsenzym und
in Relation zur Gesamtaktivität des Markierungsenzyms
so gewählt ist, daß bei gleichzeitiger Inkubation des enzymatisch markierten nicht trägerfixierten Bindungspartners mit beiden Substraten die enzymatisch katalysierte Reaktion des ein Nachweissignal erzeugenden Substrat so lange verzögert wird, bis die spezifische Bindungsreaktion zwischen den Bindungspartnern im wesentlichen abgelaufen ist.

**2.** Verfahren nach Anspruch 1, dadurch gekennzeichnet, daß der trägerfixierte Bindungspartner ein sowohl mit dem Analyten als auch mit dem enzym markierten nicht trägerfixierten Bindungspartner spezifisch bindungsfähiges Biomaterial ist.

**3.** Verfahren nach Anspruch 1, dadurch gekennzeichnet, daß der nicht trägerfixierte enzym markierte Bindungspartner ein sowohl mit dem Analyten als auch mit dem trägerfixierten Bindungspartner spezifisch bindungsfähiges Biomaterial ist.

**4.** Verfahren nach Anspruch 3, dadurch gekennzeichnet, daß dem Reaktionsschritt, bei dem der enzymatisch markierte nicht trägerfixierte Bindungspartner und der trägerfixierte Bindungspartner miteinander inkubiert werden, eine Vorreaktion vorausgeht, bei der der enzym markierte nicht trägerfixierte Bindungspartner mit dem Analyten inkubiert wird.

**5.** Verfahren nach Anspruch 3, dadurch gekennzeichnet, daß zugleich mit der Inkubation des enzym markierten nicht trägerfixierten Bindungspartners mit dem trägerfixierten Bindungspartner auch mit dem

EP 0 268 978 B1

Analyten inkubiert wird.

**6.** Verfahren nach Anspruch 1, dadurch gekennzeichnet, daß der enzym markierte nicht trägerfixierte Bindungspartner ein Antigen und der trägerfixierte Bindungspartner ein Antikörper für das Antigen ist.

**7.** Verfahren nach Anspruch 1, dadurch gekennzeichnet, daß der enzymatisch markierte nicht trägerfixierte Bindungspartner ein Antikörper und der trägerfixierte Bindungspartner ein Antigen ist, für das der Antikörper spezifisch ist.

**8.** Verfahren nach Anspruch 1, dadurch gekennzeichnet, daß zusätzlich mit dem Markierungsenzym in trägerfixierter Form inkubiert wird.

**9.** Verfahren nach Anspruch 1, dadurch gekennzeichnet, daß zusätzlich mit dem nicht trägerfixierten Bindungspartner ohne Enzymmarkierung inkubiert wird.

**10.** Verfahren nach Anspruch 1, dadurch gekennzeichnet, daß das Enzym eine höhere Affinität gegenüber dem kein Nachweissignal erzeugenden Substrat hat als gegenüber dem ein Nachweissignal erzeugenden Substrat.

**11.** Testträger für die Bestimmung eines Analyten aus einer Probe, insbesondere einer Körperflüssigkeit, mit einem Reaktionsbereich auf den die Probe aufgebracht wird, dadurch gekennzeichnet, daß der Reaktionsbereich folgende Reaktionspartner in einer derartigen Anordnung enthält, daß sie bei Benutzung des Testträgers gleichzeitig inkubiert werden:
zwei spezifisch miteinander bindungsfähige Biomaterialien (Bindungspartner) von denen der eine Bindungspartner enzymatisch markiert und löslich und
der andere Bindungspartner trägerfixiert ist.
ein nicht fixiertes, kein Nachweissignal erzeugendes Substrat des Markierungsenzyms und
ein trägerfixiertes, ein Nachweissignal erzeugendes Substrat des Markierungsenzyms,
wobei das kein Nachweissignal erzeugendes Substrat hinsichtlich eingesetzter Menge und Affinität zu dem Markierungsenzym
in Relation sur Menge des ein Nachweissignal erzeugenden Substrats und dessen Affinität zum Markierungsenzym und
in Relation zur Gesamtaktivität des Markierungsenzyms
so gewählt ist, daß bei gleichzeitiger Inkubation des ersten enzym markierten Bindungspartners mit beiden Substraten die enzymatisch katalysierte Reaktion des ein Nachweissignal erzeugenden Substrat so lange verzögert wird, bis die spezifische Bindungsreaktion zwischen dem enzym markierten Bindungspartner und dem trägerfixierten Bindungspartner im wesentlichen abgelaufen ist.

**12.** Testträger nach Anspruch 11, dadurch gekennzeichnet, daß der Reaktionsbereich das Markierungsenzyms in trägerfixierter Form und in einer solchen Anordnung enthält, daß es ebenfalls gleichzeitig inkubiert wird.

**13.** Testträger nach Anspruch 11, dadurch gekennzeichnet, daß der Reaktionsbereich den nicht trägerfixierten Bindungspartner ohne Enzymmarkierung in einer solchen Anordnung enthält, daß er ebenfalls gleichzeitig inkubiert wird.

**14.** Testträger nach Anspruch 11, dadurch gekennzeichnet, daß der Reaktionsbereich mehrere die Reationspartner enthaltene Schichten aufweist, die so angeordnet sind, daß sie bei der Benutzung des Testträgers zumindest zeitweise in einem einen Flüssigkeitsaustausch ermöglichenden Kontakt zueinander stehen.

**15.** Testträger nach Anspruch 14, dadurch gekennzeichnet, daß der trägerfixierte enzym markierte Bindungspartner in einer Schicht enthalten ist, die nicht zugleich eines der Substrate enthält.

**16.** Testträger nach Anspruch 14, dadurch gekennzeichnet, daß der enzym markierte nicht trägerfixierte Bindungspartner und der trägerfixierte Bindungspartner in getrennten Schichten enthalten sind.

**Claims**

13

1. Process for the determination of an analyte from a sample, especially a body fluid, in which two biomaterials (binding partners) capable of specifically binding with one another are used, of which one of the binding partners is enzyme-labelled and not carrier fixed and the other binding partner is carrier-fixed, which process contains a reaction step in which the binding partners are incubated with one another so that a specific binding reaction takes place between them, whereby, after ending of the specific binding reaction, the amount of the enzyme-labelled binding partner not bound to the carrier-fixed binding partner is a measure for the concentration of the analyte and this amount is determined with the help of the enzyme labelling in that one allows the labelling enzyme to act upon a substrate producing a detection signal, characterised in that, during the specific binding reaction, one incubates simultaneously with a non-fixed substrate of the labelling enzyme not producing a detection signal and with a carrier-fixed substrate of the labelling enzyme producing a detection signal, whereby the substrate not producing a detection signal is so chosen with regard to the amount used and affinity to the labelling enzyme in relation to the amount of the substrate producing a detection signal and its affinity to the labelling enzyme and in relation to the total activity of the labelling enzyme that, in the case of simultaneous incubation of the enzyme-labelled, non-carrier-fixed binding partner with both substrates, the enzyme-catalysed reaction of the substrate producing a detection signal is delayed until the specific binding reaction between the binding partners has substantially taken place.

2. Process according to claim 1, characterised in that the carrier-fixed binding partner is a biomaterial specifically bindable not only with the analyte but also with the enzyme-labelled, non-carrier-fixed binding partner.

3. Process according to claim 1, characterised in that the non-carrier-fixed, enzyme-labelled binding partner is a biomaterial specifically bindable not only with the analyte but also with the carrier-fixed binding partner.

4. Process according to claim 3, characterised in that the reaction step in which the enzyme-labelled, non-carrier-fixed binding partner and the carrier-fixed binding partner are incubated with one another is preceded by a pre-reaction in which the enzyme-labelled, non-carrier fixed binding partner is incubated with the analyte.

5. Process according to claim 3, characterised in that, simultaneously with the incubation of the enzyme-labelled non-carrier-fixed binding partner with the carrier-fixed binding partner, there is also incubated the analyte.

6. Process according to claim 1, characterised in that the enzyme-labelled, non-carrier-fixed binding partner is an antigen and the carrier-fixed binding partner is an antibody for the antigen.

7. Process according to claim 1, characterised in that the enzyme-labelled, non-carrier-fixed binding partner is an antibody and the carrier-fixed binding partner is an antigen which is specific for the antibody.

8. Process according to claim 1, characterised in that incubation is additionally carried out with the labelling enzyme in carrier-fixed form.

9. Process according to claim 1, characterised in that incubation is additionally carried out with the non-carrier-fixed binding partner without enzyme labelling.

10. Process according to claim 1, characterised in that the enzyme has a higher affinity towards the substrate not producing a detection signal than towards the substrate producing a detection signal.

11. Test carrier for the determination of an analyte from a sample, especially a body fluid, with a reaction zone to which the sample is applied, characterised in that the reaction zone contains the following reaction partners in such an arrangement that, in the case of use of the test carrier, they are simultaneously incubated: two biomaterials (binding partners) specifically bindable with one another, one of which binding partners is enzyme-labelled and soluble and the other binding partner is carrier-fixed, a non-fixed substrate of the labelling enzyme not producing a detection signal and a carrier-fixed

14

substrate of the labelling enzyme producing a detection signal, whereby the substrate not producing a detection signal is so chosen with regard to the amount used and affinity to the labelling enzyme in relation to the amount of the substrate producing a detection signal and its affinity to the labelling enzyme and in relation to the total activity of the labelling enzyme that, in the case of simultaneous incubation of the first enzyme-labelled binding partner with the two substrates, the enzyme-catalysed reaction of the substrate producing a detection signal is delayed until the specific binding reaction between the enzyme-labelled binding partner and the carrier-fixed binding partner is substantially ended.

12. Test carrier according to claim 11, characterized in that the reaction zone contains the labelling enzyme in carrier-fixed form and in such an arrangement that it is also simultaneously incubated.

13. Test carrier according to claim 11, characterised in that the reaction zone contains the non-carrier-fixed binding partner without enzyme-labelling in such an arrangement that it is also simultaneously incubated.

14. Test carrier according to claim 11, characterised in that the reaction zone has several layers containing the reaction partners which are so arranged that, in the case of use of the test carrier, they are at least for a part of time in contact with one another making possible a liquid exchange.

15. Test carrier according to claim 14, characterised in that the carrier-fixed, enzyme-labelled binding partner is contained in a layer which does not simultaneously contain one of the substrates.

16. Test carrier according to claim 14, characterised in that the enzyme-labelled, non-carrier-fixed binding partner and the carrier-fixed binding partner are contained in separate layers.

**Revendications**

1. Procédé de détermination d'un analyte à partir d'un échantillon, en particulier d'un liquide corporel,

dans lequel sont utilisés deux biomatériaux (partenaires de liaison) capables de se lier l'un à l'autre de manière spécifique,

dont l'un des partenaires de liaison est marqué de manière enzymatique et n'est pas fixé au support, et

l'autre partenaire de liaison est fixé au support,

procédé qui comporte une étape de réaction, au cours de laquelle les partenaires de liaison sont incubés entre eux, de telle façon qu'il se produise une réaction de liaison spécifique entre eux, d'où il résulte qu'après le déroulement de la réaction de liaison spécifique, la quantité du partenaire de liaison, marqué enzymatiquement et non lié avec le partenaire de liaison fixé sur le support, est une mesure pour la concentration de l'analyte, et que cette quantité est déterminée au moyen du marquage enzymatique, tandis qu'on laisse agir l'enzyme de marquage sur un substrat produisant un signal indicateur,

caractérisé en ce que,

pendant la réaction de liaison spécifique, en même temps avec un substrat de l'enzyme de marquage, non fixé et ne produisant aucun signal indicateur, on incube un substrat de l'enzyme de marquage fixé au support et

produisant un signal indicateur,

d'où il résulte qu'on choisit le substrat ne produisant aucun signal indicateur, en ce qui concerne la quantité et l'affinité par rapport à l'enzyme de marquage, en fonction de la quantité du substrat produisant un signal indicateur et de son affinité par rapport à l'enzyme de marquage, et

en relation avec l'activité totale de l'enzyme de marquage,

de telle façon qu'avec une incubation simultanée du partenaire de liaison non fixé au support et marqué enzymatiquement avec les deux substrats, la réaction, catalysée enzymatiquement, du substrat produisant un signal indicateur est retardée assez longtemps pour que la réaction de liaison spécifique entre les partenaires de liaison soit essentiellement complète.

2. Procédé suivant la revendication 1, caractérisé en ce que le partenaire de liaison fixé au support est un biomatériau susceptible de se lier de façon spécifique aussi bien avec l'analyte qu'avec le partenaire de liaison non fixé au support et marqué par un enzyme.

3. Procédé suivant la revendication 1, caractérisé en ce que le partenaire de liaison non fixé sur le support et marqué par un enzyme est un biomatériau susceptible de se combiner de façon spécifique aussi bien avec l'analyte qu'avec le partenaire de liaison fixé au support.

4. Procédé suivant la revendication 3, caractérisé en ce qu'au stade de la réaction où le partenaire de liaison non fixé sur le support et marqué par un enzyme et le partenaire de liaison fixé sur le support ont été incubés l'un avec l'autre, il en résulte une réaction préalable par laquelle le partenaire de liaison non fixé sur le support et marqué par un enzyme est incubé avec l'analyte.

5. Procédé suivant la revendication 3, caractérisé en ce que l'on procède en même temps à l'incubation du partenaire de liaison non fixé sur le support et marqué par un enzyme avec le partenaire de liaison fixé sur le support ainsi qu'avec l'analyte.

6. Procédé suivant la revendication 1, caractérisé en ce que le partenaire de liaison non fixé sur le support et marqué par un enzyme est un antigène et que le partenaire de liaison fixé sur le support est un anticorps pour l'antigène.

7. Procédé suivant la revendication 1, caractérisé en ce que le partenaire de liaison non fixé sur le support et marqué par un enzyme est un anticorps et que le partenaire de liaison fixé sur le support est un antigène spécifique de l'anticorps.

8. Procédé suivant la revendication 1, caractérisé en ce qu'on procède, en outre, à l'incubation avec l'enzyme de marquage sous la forme fixée au support.

9. Procédé suivant la revendication 1, caractérisé en ce qu'on procède, en outre, à l'incubation avec le partenaire de liaison non fixé au support, sans marquage avec un enzyme.

10. Procédé suivant la revendication 1, caractérisé en ce que l'enzyme a une affinité plus grande par rapport au substrat ne produisant aucun signal indicateur que par rapport au substrat produisant un signal indicateur.

11. Support de test pour la détermination d'un analyte constitué d'un échantillon, en particulier d'un liquide corporel, comportant une zone de réaction sur laquelle l'échantillon est déposé, caractérisé en ce que la zone de réaction comporte des partenaires de liaison dans un ordre tel qu'ils soient incubés simultanément lors de l'utilisation du support de test :
   deux biomatériaux (partenaires de liaison) susceptibles de se combiner entre eux de façon spécifique, dont l'un est un partenaire de liaison marqué enzymatiquement et soluble, et
   l'autre partenaire de liaison est fixé sur le support,
   un substrat de l'enzyme de marquage, non fixé et ne produisant pas de signal indicateur,
   un substrat de l'enzyme de marquage, fixé au support et produisant un signal indicateur,
   d'où il résulte que le substrat qui ne produit pas de signal indicateur est choisi, en ce qui concerne la quantité et l'affinité par rapport à l'enzyme de marquage,
   en relation avec la quantité du substrat produisant un signal indicateur et avec son affinité par rapport à l'enzyme de marquage, et
   en relation avec l'activité totale de l'enzyme de marquage,
   de telle façon qu'avec une incubation simultanée du premier partenaire de liaison marqué par un enzyme, avec les deux substrats, la réaction enzymatiquement catalysée, du substrat produisant le signal indicateur est retardée assez longtemps pour que la réaction de combinaison spécifique entre le partenaire de liaison marqué par un enzyme et le partenaire de liaison fixé au support soit essentiellement complète.

12. Support de test suivant la revendication 11, caractérisée en ce que la zone de réaction contient l'enzyme de marquage sous une forme fixée sur le support et dans un ordre tel qu'il soit, de la même façon, incubé simultanément.

13. Support de test suivant la revendication 11, caractérisé en ce que la zone de réaction contient le partenaire de liaison non fixé au support, sans marquage par un enzyme, dans un ordre tel qu'il soit, de la même façon, incubé simultanément.

14. Support de test suivant la revendication 11, caractérisé en ce que la zone de réaction présente plusieurs couches disposées de façon à être, lors de l'utilisation du support de test, au moins une partie du temps, l'une avec l'autre, en contact permettant un échange de liquides.

15. Support de test suivant la revendication 14, caractérisé en ce que le partenaire de liaison marqué par un enzyme et fixé au support est contenu dans une couche qui ne contient pas en même temps l'un des substrats.

16. Support de test suivant la revendication 14, caractérisé en ce que le partenaire de liaison non fixé au support et marqué par un enzyme et le partenaire de liaison fixé au support sont contenus dans des couches différentes.

Fig.1

Fig.2

Fig.3